(19) Europäisches Patentamt
European Patent Office
Office européen des brevets

(11) Veröffentlichungsnummer: **0 022 462**
**B1**

(12) # EUROPÄISCHE PATENTSCHRIFT

(45) Veröffentlichungstag der Patentschrift: **25.11.81**

(21) Anmeldenummer: **80102772.3**

(22) Anmeldetag: **20.05.80**

(51) Int. Cl.³: **C 07 C 43/184,** C 07 C 43/115, C 07 C 43/162, C 11 B 9/00, A 61 K 7/46

(54) **2-Alkoxiethyl-cycloalkyl-ether, diese enthaltende Stoffkombinationen sowie ihre Verwendung zur Herstellung von Riechstoffkompositionen.**

(30) Priorität: **13.07.79 DE 2928348**

(43) Veröffentlichungstag der Anmeldung: **21.01.81 Patentblatt 81/3**

(45) Bekanntmachung des Hinweises auf die Patenterteilung: **25.11.81 Patentblatt 81/47**

(84) Benannte Vertragsstaaten: **CH FR GB IT LI**

(56) Entgegenhaltungen:
**DE-A1-2 427 500**
**DE-A1-2 436 520**
**DE-B2-2 626 965**

(73) Patentinhaber: **CHEMISCHE WERKE HÜLS AG, Postfach 1320, D-4370 Marl 1 (DE)**

(72) Erfinder: **Burzin, Klaus, Dr., Wellerfeldweg 164, D-4370 Marl (DE)**

**EP 0 022 462 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## 2-Alkoxiethyl-cycloalkyl-ether, diese enthaltende Stoffkombinationen sowie ihre Verwendung zur Herstellung von Riechstoffkompositionen

Es wurde gefunden, daß aliphatische Ether der allgemeinen Formel

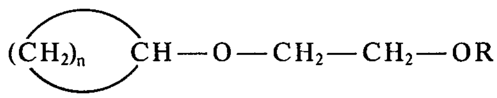

in der R ein geradkettiger oder verzweigter gesättigter oder ungesättigter Alkylrest mit 1 bis 3 Kohlenstoffatomen und n eine ganze Zahl von 5 bis 11 ist, neue Riechstoffe mit einer holzartigen Geruchsnote und fixierenden Eigenschaften darstellen.

Die Herstellung der erfindungsgemäßen Ether erfolgt gewöhnlich durch Veretherung der 2-Hydroxyethyl-cycloalkylether. Dabei können die bekannten Veretherungsverfahren angewendet werden (siehe Houben—Weyl, Methoden der organischen Chemie, Band VI/3, Sauerstoffverbindungen I, Teil 3, Seiten 10 bis 137 [1965]).

Die Reinigung der Rohether erfolgt in der Regel durch fraktionierte Destillation. Die Entfernung letzter Reste nicht veretherter Alkohole ist auch durch andere Trennverfahren, wie z. B. Extraktions- oder Absorptionsverfahren möglich.

Die 2-Hydroxiethyl-cycloalkyl-ether lassen sich prinzipiell durch Ethoxilierung der entsprechenden Cycloalkanole mit Ethylenoxid bzw. mit Ethylencarbonat unter Einhaltung der für die Ethoxilierung üblichen Reaktionsbedingungen herstellen. Dabei empfiehlt es sich zwecks Erhöhung der Acidität der sekundären Hydroxylgruppe entsprechend dem Verfahren der DE-PS 5 974 767 Bortrifluorid hinzuzusetzen. Dabei kannn jedoch nicht verhindert werden, daß der Ethoxilierungsgrad auch $>1$ ist und daß gleichzeitig Polyethylenoxid gebildet wird.

Wesentlich selektiver ist dagegen die Herstellung der 2-Hydroxiethyl-cycloalkyl-ether durch Hydrierung der aus den Cycloalkanonen und Ethylenglykol bereiteten Dioxolane, wie sie in der GB-PS 1 125 730 beschrieben ist.

Unter den erfindungsgemäßen Ethern kommt den Substanzen mit $n=5$, 7 und 11, $R=CH_3$ und $C_2H_5$ die größte Bedeutung zu, wobei die Substanzen mit $R=CH_3$ bevorzugt sind. Andere mögliche Reste R sind der Propyl-, Isopropyl-, Allyl- und Propargylrest.

Typische Vertreter der erfindungsgemäßen Ether sind der 2-Methoxiethyl-cyclohexyl-ether, der 2-Ethoxiethyl-cyclohexyl-ether, der 2-Methoxiethyl-cyclooctyl-ether, der 2-Ethoxiethyl-cyclooctyl-ether, der 2-Methoxiethyl-cyclododecyl-ether und der 2-Ethoxiethyl-cyclododecyl-ether.

Mit anderen Stoffen, vornehmlich mit anderen Riechstoffen, können die erfindungsgemäßen Ether in den verschiedensten Mengenverhältnissen zu neuen Stoffkombinationen bzw. neuen Riechstoffkompositionen vermischt werden. Dabei beträgt der Anteil der beanspruchten Ether im allgemeinen bis zu 60, vorzugsweise 1 bis 50 Gewichtsprozent.

Die Riechstoffkompositionen können als Parfüm oder zur Parfümierung von Kosmetika, wie z. B. Cremes, Feinseifen und Lotionen dienen. Außerdem können sie zur Geruchsverbesserung technischer Produkte, wie z. B. Wasch- und Reinigungsmittel, Desinfektionsmittel und Textilhilfsmittel verwendet werden.

Die nachfolgenden Beispiele sollen den Gegenstand der vorliegenden Erfindung näher erläutern, ohne ihn jedoch hierauf zu beschränken, da sich die übrigen aliphatischen Ether auf entsprechende Weise herstellen lassen und ebenfalls die typische holzartige Geruchsnote besitzen.

### Beispiel 1

### 2-Methoxiethyl-cyclododecyl-ether

25 g Natriumamid wurden in 150 ml Xylol eingebracht und unter Rühren bis zum Sieden erhitzt. Innerhalb einer Stunde ließ man 114 g (0,5 Mol) 2-Hydroxiethyl-cyclododecyl-ether, gelöst in 750 ml Xylol, in die siedende Suspension tropfen. Zur Vervollständigung der Alkoholatbildung wurde weitere zwei Stunden am Rückfluß erhitzt. Danach wurden tropfenweise 44 g (0,35 Mol) Dimethylsulfat hinzugefügt und das Reaktionsgemisch weitere vier Stunden rückfließend erhitzt. Anschließend wurde das Reaktionsgemisch in einer Mischung aus Eis und 35 g Natriumhydroxid gegossen. Die organische Phase wurde mit Wasser gewaschen, über $Na_2SO_4$ getrocknet und danach fraktioniert.

| | |
|---|---|
| Siedepunkt | 105° C bei 0,05 mbar |
| Ausbeute | 84% Rohether, 66% Reinprodukt |
| Brechungsindex $n_D^{20}$ | 1,4713 |
| IR | 1100 $cm^{-1}$ |
| NMR ($CCl_4$) | $\delta$ 3,40 (S) ($-O-CH_2-CH_2-O$), $\delta$ 3,25 (S) ($-O-CH_3$) |

Beispiel 2

2-Ethoxiethyl-cyclododecyl-ether

Der 2-Ethoxiethyl-cyclododecyl-ether wurde entsprechend den Angaben in Beispiel 1 aus 114 g (0,5 Mol) 2-Hydroxiethyl-cyclododecyl-ether und 54 g (0,35 Mol) Diethylsulfat hergestellt.

| | |
|---|---|
| Siedepunkt | 109° C bei 0,05 mbar |
| Ausbeute | 89% Rohether, 72% Reinprodukt |
| Brechungsindex $n_D^{20}$ | 1,4688 |
| NMR ($CCl_4$) | $\delta$ 3,38 (Q) ($-O-CH_2-CH_3$), |
| | $\delta$ 3,40 (S) ($-O-CH_2-CH_2-O-$) |

Beispiel 3

2-Methoxiethyl-cyclooctyl-ether

Nach den Angaben in Beispiel 1 wurde der 2-Methoxiethyl-cyclooctyl-ether durch Umsetzung von 86 g (0,5 Mol) 2-Hydroxiethyl-cyclooctyl-ether mit 44 g (0,35 Mol) Dimethylsulfat gewonnen.

| | |
|---|---|
| Siedepunkt | 58° C bei 0,1 mbar |
| Ausbeute | 86% Rohether, 69% Reinprodukt |
| Brechungsindex $n_D^{20}$ | 1,4617 |
| IR | 1100 $cm^{-1}$ |
| NMR ($CCl_4$) | $\delta$ 3,42 (S) ($-O-CH_2-CH_2-O$), |
| | $\delta$ 3,25 (S) ($-O-CH_3$) |

Beispiel 4

2-Ethoxiethyl-cyclooctyl-ether

Der 2-Ethoxiethyl-cyclooctyl-ether wurde entsprechend den Angaben in Beispiel 1 durch Umsetzung von 86 g (0,5 Mol) 2-Hydroxiethyl-cyclooctyl-ether mit 54 g (0,35 Mol) Diethylsulfat hergestellt.

| | |
|---|---|
| Siedepunkt | 84° C bei 0,5 mbar |
| Ausbeute | 94% Rohether, 75% Reinprodukt |
| Brechungsindex $n_D^{20}$ | 1,4583 |
| IR | 1100 $cm^{-1}$ |
| NMR ($CCl_4$) | $\delta$ 3,42 (S) ($-O-CH_2-CH_2-O$), |
| | $\delta$ 3,39 (Q) ($-O-CH_2-CH_3$) |

Beispiel 5

2-Methoxiethyl-cyclohexyl-ether

Nach der in Beispiel 1 beschriebenen Weise wurde der 2-Methoxiethyl-cyclohexyl-ether aus 72 g (0,5 Mol) 2-Hydroxiethyl-cyclohexyl-ether und 44 g (0,35 Mol) Dimethylsulfat hergestellt.

| | |
|---|---|
| Siedepunkt | 46° C bei 0,4 mbar |
| Ausbeute | 88% Rohether, 76% Reinprodukt |
| Brechungsindex $n_D^{20}$ | 1,4428 |
| IR | 1100 $cm^{-1}$ |
| NMR ($CCl_4$) | $\delta$ 3,42 (S) ($-O-CH_2-CH_2-O$), |
| | $\delta$ 3,25 (S) ($-O-CH_3$) |

## Beispiel 6

### 2-Ethoxiethyl-cyclohexyl-ether

Der 2-Ethoxiethyl-cyclohexyl-ether wurde entsprechend den Angaben in Beispiel 1 aus 72 g (0,5 Mol) 2-Hydroxiethyl-cyclohexyl-ether und 54 g (0,35 Mol) Diethylsulfat hergestellt.

| | |
|---|---|
| Siedepunkt | 46° C bei 0,1 mbar |
| Ausbeute | 87% Rohether, 71% Reinprodukt |
| Brechungsindex $n_D^{20}$ | |
| IR | 1100 $cm^{-1}$ |
| NMR ($CCl_4$) | $\delta$ 3,43 (S) ($-O-CH_2-CH_2-O$), <br> $\delta$ 3,41 (Q) ($-O-CH_2-CH_3$) |

Nachfolgend werden Beispiele für Riechstoffkompositionen angegeben, die erfindungsgemäße Substanzen enthalten.

## Beispiel 7

### (Holznote)

| | Gew.-Teile |
|---|---|
| 2-Methoxiethyl-cyclododecyl-ether | 250 |
| Sandelholzöl | 350 |
| Bergamottöl | 100 |
| Vetiveröl | 50 |
| Amylsalicylat | 80 |
| Cumarin | 50 |
| Rosenöl | 50 |
| Heliotropin | 50 |
| Xylolmoschus | 20 |
| | 1000 |

## Beispiel 8

### (Holznote)

| | Gew.-Teile |
|---|---|
| 2-Ethoxiethyl-cyclododecyl-ether | 500 |
| Sandelholzöl | 100 |
| Vetiverylacetat | 100 |
| Oryclon | 100 |
| Cumarin | 50 |
| Guajylacetat | 50 |
| 2-Phenylethanol | 50 |
| Isoraldein 70 | 50 |
| | 1000 |

Beispiel 9

(Phantasie-Parfümierung)

| | Gew.-Teile |
|---|---|
| 2-Ethoxiethyl-cyclooctyl-ether | 350 |
| Anisaldehyd | 150 |
| Lavendelöl | 100 |
| Geraniumöl | 100 |
| Zedernöl | 100 |
| Butylphenylacetat | 50 |
| Hydroxicitronellal | 50 |
| Methylnaphthylketon | 50 |
| Benzylacetat | 35 |
| Xylolmoschus | 15 |
| | 1000 |

In den beispielhaft aufgeführten Riechstoffkompositionen können anstelle der genannten 2-Alkoxiethyl-cycloalkyl-ether auch die anderen erfindungsgemäßen Verbindungen eingesetzt werden, wodurch sich allerdings Verschiebungen in den Geruchsnuancen ergeben.

**Patentansprüche**

1. 2-Alkoxiethyl-cycloalkyl-ether der allgemeinen Formel

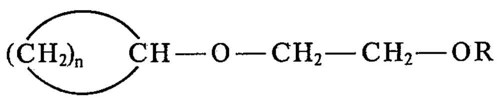

in der R ein geradkettiger oder verzweigter gesättigter oder ungesättigter Alkylrest mit 1 bis 3 Kohlenstoffatomen und n eine ganze Zahl von 5 bis 11 ist.

2. 2-Alkoxiethyl-cycloalkyl-ether nach Anspruch 1, dadurch gekennzeichnet, daß n = 5, 7 oder 11 ist.

3. 2-Alkoxiethyl-cycloalkyl-ether nach Anspruch 2, dadurch gekennzeichnet, daß n = 5 und R = $CH_3$, $C_2H_5$ oder i-$C_3H_7$ ist.

4. 2-Alkoxiethyl-cycloalkyl-ether nach Anspruch 2, dadurch gekennzeichnet, daß n = 7 und R = $CH_3$, $C_2H_5$, i-$C_3H_7$, $CH_2-CH=CH_2$ oder $CH=CH-CH_3$ ist.

5. 2-Alkoxiethyl-cycloalkyl-ether nach Anspruch 2, dadurch gekennzeichnet, daß n = 11 und R = $CH_3$ oder $C_2H_5$ ist.

6. Stoffkombination, gekennzeichnet durch einen Gehalt an einer Verbindung nach Anspruch 1.

7. Verwendung der Verbindungen nach Anspruch 1 zur Herstellung von Riechstoffkompositionen.

**Claims**

1. 2-alkoxyethyl-cycloalkyl-ether, the general formula being

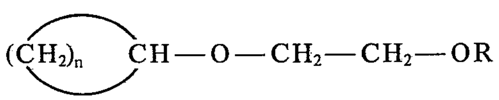

wherein R is a straight-chain or branched saturated or unsaturated alkyl radical having 1 to 3 carbon atoms, and n a whole number from 5 to 11.

2. 2-alkoxyethyl-cycloalkyl-ether according to Claim 1, characterized in that n = 5, 7 or 11.

3. 2-alkoxyethyl-cycloalkyl-ether according to Claim 2, characterized in that n = 5 and R = $CH_3$, $C_2H_5$ or i-$C_3H_7$.

4. 2-alkoxyethyl-cycloalkyl-ether according to Claim 2, characterized in that n = 7 and R = $CH_3$, $C_2H_5$, i-$C_3H_7$, $CH_2-CH=CH_2$ or $CH=CH-CH_3$.

5. 2-alkoxyethyl-cycloalkyl-ether according to Claim 2, characterized in that n = 11 and R = $CH_3$ or $C_2H_5$.

6. A compound, characterized in that it contains a substance according to Claim 1.

7. Use of the substances according to Claim 1 for the purpose of producing perfumes.

**Revendications**

1. 2-alkoxyéthyle-cycloalcoyle-éther de la formule générale

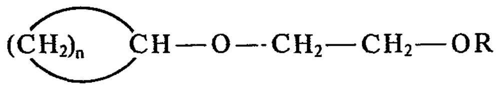

dans laquelle R est un radical alcoyle à chaîne droite ou ramifié, saturé ou non saturé, avec 1 à 3 atomes de carbone et n est un nombre entier de 5 à 11.

2. 2-alkoxyéthyle-cycloalcoyle-éther suivant la revendication 1, caractérisé par le fait que n = 5, 7 ou 11.

3. 2-alkoxyéthyle-cycloalcoyle-éther suivant la revendication 2, caractérisé par le fait que n = 5 et R = $CH_3$, $C_2H_5$ ou est i-$C_3H_7$.

4. 2-alkoxyéthyle-cycloalcoyle-éther suivant la revendication 2, caractérisé par le fait que n = 7 et R = $CH_3$, $C_2H_5$, i-$C_3H_7$, $CH_2-CH=CH_2$ ou est $CH=CH-CH_3$.

5. 2-alkoxyéthyle-cycloalcoyle-éther suivant la revendication 2, caractérisé par le fait que n = 11 et R = $CH_3$ ou est $C_2H_5$.

6. Combinaison de substances, caractérisé par une teneur en une combinaison suivant la revendication 1.

7. Utilisation des combinaisons suivant la revendication 1 pour fabriquer des parfums.